# EUROPEAN PATENT APPLICATION

(11) **EP 0 744 470 A1**
(43) Date of publication of application: **27.11.1996**
(21) Application number: 96303584.5
(22) Date of filing: 21.05.1996
(51) Int. Cl.: C12Q 1/68, C12P 19/34, C07K 16/40, C12Q 1/44

(54) **Methods for polymerase chain reaction preamplification sterilization by exonucleases in the presence of phosphorothioated primers**

(30) Priority: 22.05.1995 US 447000
(71) Applicant: JOHNSON & JOHNSON CLINICAL DIAGNOSTICS, INC., Rochester, New York 14650-0880 (US)
(72) Inventor: Backus, John Wesley, Williamson, New York 14589 (US); Patterson, David Robert, Rochester, New York 14607 (US); Sutherland, John William Henderson, Williamson, New York 14620 (US)
(74) Representative: Mercer, Christopher Paul

(57) **Abstract**

The present invention provides admixtures and methods for PCR amplification of a target nucleic acid in which amplification efficiency is increased by including phosphorothioated oligonucleotide primers and an exonuclease in the PCR reaction mix. Kite for amplification of a target nucleic acid are also provided.

## Description

### FIELD OF THE INVENTION

Polymerase chain reaction (PCR) allows amplification and detection of small quantities of a target nucleic acid. Practical limitations of PCR include contamination by amplified nucleic acids carried over from a previous reaction, leading to false positive results, and the production and amplification of other non-specific products, such as primer-dimers, leading to false negative results. The present invention overcomes such limitations by providing a method of PCR utilizing phosphorothioated primers and preamplification sterilization in the presence of an exonuclease.

### BACKGROUND OF THE IHVENTION

The technology of PCR permits amplification and subsequent detection of minute quantities of a target nucleic acid. Details of PCR are well described in the art, including, for example, U.S. Patent Nos. 4,683,195 to Mullis et al., 4,683,202 to Mullis and 4,965,188 to Mullis et al. Generally, oligonucleotide primers are annealed to the denatured strands of a target nucleic acid, and primer extension products are formed by the polymerization of deoxynucleoside triphosphates by a polymerase. A typical PCR method involves repetitive cycles of template nucleic acid denaturation, primer annealing and extension of the annealed primers by the action of a thermostable polymerase. The process results in exponential amplification of the target nucleic acid, and thus allows the detection of targets existing in very low concentrations in a sample.

PCR is widely used in a variety of applications, including biotechnological research, clinical diagnostics and forensics. However, the methodology is subject to practical limitations that result in less than optimal efficiency and specificity. In particular, before the first cycle of a PCR experiment (i.e., at "zero cycle"), the reagents for amplification are typically mixed and stored at room temperature or lower. Because thermostable polymerases, for example, Thermus aquaticus (Taq) polymerase, have residual activity even at 0°C, relatively large quantities of non-specific products can be formed by low stringency priming and extension. The non-specific products, known as zero cycle artifacts, include primer-dimers formed by ligation of primers having homology at their 3' ends. Because of the micromolar concentrations of primers used in PCR relative to the often minute concentrations of target, the formation of primer-dimers may be predominant. Primer-dimers are thus particularly pervasive zero-cycle artifacts. Other primer based amplification systems, such as solid phase amplification, similarly suffer from primer-dimer artifacts.

The formation of zero-cycle artifacts during amplification has practical consequences. Reagents, including primers and deoxyribonucleosides, may be depleted, and the non-specific side products act as competitive inhibitors with respect to the target for the polymerase and other limiting components of the reaction. Consequently, amplification efficiency may be decreased and assay precision degraded. Likewise, presence of primer-dimer due to carryover from a previously amplified sample would also be detrimental. Any decrease in amplification efficiency may adversely effect the assay detection limit, and thus potentially cause false negative results. Primer-dimer formation can reduce efficiency of target amplification to such a degree that the amplified product is not detectable on a stained gel. Such a result would clearly be undesirable in tests for pathogens, such as HIV.

Specificity is particularly important in homogeneous PCR reactions. See, e.g., EPA 487218 to Mitoma; EPA 512334 to Higuchi. In the homogeneous assays, PCR amplification and detection are coupled by contacting the reaction mixture, during or after amplification, with a fluorescent pigment that undergoes a detectable change in fluorescence upon reaction with a double-stranded nucleic acid. For example, when PCR is conducted in the presence of ethidium bromide, the production of double-stranded DNA is correlated with an increase in fluorescence as free ethidium bromide becomes intercalated into double-stranded DNA. Generally, amplification and detection are carried out in the same vessel. Changes in fluorescence are detected spectrophotometrically, and thus detection requires neither separation of PCR products nor hybridization. Because detection is based upon formation of double-stranded DNA generally, and fails to discriminate between target DNA and non-specific products, the formation of double-stranded artifacts such as primer-dimers is fatal to the specificity of the homogeneous assay.

Another significant practical limitation of PCR results from contamination by exogenous DNA in the laboratory environment or by carryover of previously amplified DNA (amplicons) that can serve as templates in later amplifications. Amplicon carryover commonly leads to false-positive results, while primer-dimer carryover leads to false negative results.

Various strategies have been developed to overcome these limitations and thus increase PCR specificity and efficiency. Theoretically, primer-dimer artifacts can be minimized by selecting primers with no 3'-complementarity. In practice, however, some 3'-complementarity may be unavoidable, particularly in applications that require mixtures of primers. Coamplification of numerous strains or alleles of a target are typical applications that require a large number of primers.

Specificity can also be improved by increasing stringency, for example, by increasing the annealing temperature or incorporating denaturing solvents. However, increasing stringency may lead to false negative results because the assay's ability to detect mutated forms of the target, which may have been amplified at lower stringency, is reduced.

In another method of reducing zero-cycle artifacts, the so-called "hot start" method of PCR, the reaction is started by the addition of polymerase to hot reagent mixtures. (See, e.g., Erlich et al. (1991) Science 252:1642). Primer-dimers are reduced since the reactive intermediates formed by cross-reaction of primers are thermally unstable. However, this method does not provide the convenience of room temperature preparation, and is subject to complications caused by timing errors resulting from manual addition of polymerase to multiple (typically 96) PCR tubes. In addition, "hot starts" may result in increased likelihood of carryover contamination due to amplicons and/or primer-dimers because tubes must be opened for addition of enzyme.

Thermolabile physical barriers, such as paraffin beads or overlays, have been used to physically separate one or more PCR components from the others until temperatures suitable for high stringency priming are reached (see, e.g., H6bert et al. (1993) Molecular and Cellular Probes 7:249). However, these methods are generally inconvenient and require considerable manual dexterity.

Thermally labile antibodies to Taq polymerase have been used to inhibit Taq polymerase at low temperatures in an attempt to limit zero-cycle artifacts. (See, e.g., Sharkey et al. (1994) Bio/Technology 12:506; U.S. Patent No. 5,338,671 to Scalice et al.) When the temperature is elevated in the PCR thermal cycling, the antibodies are thermally denatured and active polymerase is released. However, even avid antibodies do not completely inhibit polymerase activity. For example, one micromolar antibody having affinity of 10¹⁰M⁻¹ acting on polymerase at a concentration of 10 nanomolar in a volume of 100 microliters would leave 60 million molecules of free active polymerase at equilibrium. Since primer levels used in PCR are relatively large, sizable number of primer-dimer intermediates can nonetheless be formed and amplified

Various preamplification sterilization procedures have been developed to minimize amplicon carryover. The enzyme uracil-N-glycosylase (UNG) has been used in a preamplification step to cleave products made during the zero cycle at incorporated uracil residues. (See, e.g., Longo et al. (1990) Gene 93:125; Espy et al. (1993) J. Clin. Microbiol. 31:2361.) Deoxyuridine triphosphate (dUTP) is substituted for deoxythymidine triphosphate (dTTP) in the PCR and thus PCR products can be distinguished from template DNA. The enzyme UNG is included in the premix, and catalyzes the excision of uracil from single or double-stranded DNA present in the reaction prior to the first PCR cycle. The resulting abasic polynucleotides are susceptible to hydrolysis and cannot function as templates during PCR. While UNG is reportedly inactivated by thermal denaturation, residual activity may degrade amplification products synthesized during PCR. Further, Longo et al. compared the relative amount of amplification product in the presence and absence of UNG treatment, and reported a reduction in the intensity of the amplified target in reactions with UNG treatment. Thus UNG treatment would not be expected to solve the problem of inefficiency of product amplification. Further, UNG is reportedly inefficient at attacking amplicons of less than 100 base pairs, for example primer-dimers (Espy et al.).

Preamplification sterilization with exonucleases has also been reported. Muralidhar et al. (1992) Gene 117:107 disclose that phage T7 exonuclease can inactivate PCR carryover product molecules but it reportedly leaves genomic DNA targets intact. The contaminating amplicons are reportedly preferentially inactivated due to their symmetric geometry relative to genomic target molecules. Zhu et al. (1991) Nucleic Acids Res. 19:251) report the use of exonuclease III (Exo III) for pre-PCR sterilization to reduce amplicon and primer-dimer carryover. Exo III catalyzes the sequential cleavage of 5 mononucleotides from the 3' hydroxyl end of duplex DNA and also reportedly leaves genomic DNA largely intact due to the length of the flanking DNA sequence.

The use of exonucleases such as Exo III for preamplification sterilization is based upon the reported specificity of the exonucleases for double-stranded DNA. Particularly with regard to Exo III, the prior art is clear that Exo III does not degrade single-stranded target DNA. See, e.g., Zhu et al.; Promega Catalog, 1994, 136. Any single-stranded exonuclease activity would degrade single primer strands, thus reducing amplification efficiency and increasing the possibility of false negative results.

It has been discovered in accordance with the present invention that preamplification sterilization with exonucleases such as Exo III as described by Zhu et al. results in a reduction of signal from low level genomic targets. The consequence of the reduction in signal is an increased possibility of false negative results, with attendant serious implications, particularly in the diagnosis of disease. Loss of target signal has been ascribed specifically to genomic target degradation. It has been discovered in accordance with the present invention that the reduction in efficiency of amplification is due to degradation of single-stranded primers, contrary to the teachings of the art regarding the double stranded specificity of Exo III. The present invention thus provides the identification of a previously unrecognized problem, and further provides a solution to the problem and thus a method of increasing efficiency of PCR.

### SUMMARY OF THE INVENTION

The present invention is directed to a method for the amplification of a target nucleic acid comprising contacting a sample suspected of containing a target nucleic acid with at least two oligonucleotide primers that are sufficiently complementary to regions of the target nucleic acid to hybridize thereto, wherein each of the oligonucleotide primers contains at least one phosphorothioate linkage, at least four different nucleoside triphosphates, a thermostable polymerization agent, and an exonuclease to form a reaction admixture; heating the reaction admixture to inactivate the exonuclease; and forming primer extension products.

The present invention further provides a kit for amplification of a target nucleic acid comprising, in the same or separate containers, a thermostable polymerization agent, an exonuclease, and at least two primers that are sufficiently complementary to regions of the nucleic acid to hybridize thereto, wherein each of the oligonucleotide primers contains at least one phosphorothioate linkage.

In another embodiment, the present invention provides an admixture for use in PCR comprising an exonuclease and at least two oligonucleotide primers wherein each of the oligonucleotide primers contains at least one phosphothioate linkage. The admixture may also contain a thermostable polymerization agent, nucleoside triphosphates, a monoclonal antibody against the polymerization agent, and other reagents for PCR.

### DETAILED DESCRIPTION OF THE INVENTION

The present invention provides a method for the amplification of a target nucleic acid whereby carryover contamination of amplicons and primer-dimers and other zero-cycle artifacts is reduced relative to conventional methods of PCR without compromise of amplification efficiency. In particular, the present method comprises the use of an exonuclease in a preamplification step to degrade carryover DNA and zero-cycle artifacts, and the use of phosphorothioated primers to avoid the heretofore unexpected primer degradation by the exonuclease.

The principles of PCR and the conditions for amplification and detection of target nucleic acids are well known in the art and may be found in numerous references known to the skilled artisan, including, for example, United States Patent Nos. 4,683,195 to Mullis et al., 4,683,202 to Mullis et al. and 4,965,188 to Mullis et al. Briefly, a sample suspected of containing a target nucleic acid is heated to denature double-stranded nucleic acid in the presence of two oligonucleotide primers that are complementary to target sequences flanking the region to be amplified. The primers anneal to the separated target strands and are extended from each 3' hydroxyl end by a polymerizing agent such as a thermostable polymerase. Double-stranded or single-stranded DNA can be amplified by PCR. RNA can also serve as a target by reverse transcribing RNA into cDNA. The steps of denaturation, primer annealing and DNA synthesis are carried out at discrete temperatures, and repeated cycles result in exponential accumulation of the target nucleic acid. The PCR vessel is generally a stoppered plastic vessel or a cuvette or pouch as described in U.S. Patent No. 5,229,297. Reagents for PCR amplification are typically mixed in a single vessel, and generally include primers, nucleoside triphosphates (generally dATP, dCTP, dGTP and dTTP or dUTP), thermostable DNA polymerase, magnesium containing buffer, and target nucleic acid. Reagents and conditions for PCR are well-known to one of ordinary skill in the art, and can be found, for example, in Guatelli et al. (1989) Clin. Microbiol. Rev. 2:217. For amplification of RNA targets, a reverse transcriptase may be utilized in addition to or in lieu of the thermostable DNA polymerase. Thermostable reverse transcriptase are particularly useful, as are thermostable DNA polymerases having reverse transcriptase activity. Methods for PCR amplification of RNA targets are known to one of ordinary skill in the art and described, for example, in U.S. Patent Nos. 5,176,995, 5,310,652 and 5,322,770. The present invention is also useful in other primer based methods of nucleic acid amplification, for example LCR and gap LCR described in International Patent Publication WO 9300447.

The present invention provides a modification of known methods of nucleic acid amplification in order to improve the efficiency of amplification of a target nucleic acid and to reduce false negative and false positive results. In particular, the methods of the present invention reduce the formation of zero cycle artifacts including primer-dimers with a concurrent increase in the efficiency of amplification of target DNA. Primer-dimers may be double-stranded PCR products consisting of the two primers and their complementary sequence. Additional bases may be inserted between the primers. (Erlich et al., (1991) Science 252:1643). Primer-dimer formation is particularly favored when primers have complementarity at the 3' ends, and may result in reduced amplification efficiency to such a degree that amplified target cannot be detected, leading to false negative results. Another limitation of known methods of PCR that is overcome by the methods of the present invention is amplification of carryover contaminants. PCR samples may be contaminated by amplicons, or amplifiable product molecules, or primer-dimer molecules, produced in a prev-ious PCR. The amplification of such molecules commonly leads to false results.

The method of the present invention thus allows a reduction in false negative and false positive results and an increase in efficiency of amplification.

In accordance with the method of the present invention, preamplification sterilization is performed in the presence of a 3' exonuclease. Exonucleases that preferentially degrade double stranded DNA are preferred. The preamplification sterilization serves to degrade duplex artifacts such as primer-dimers, as well as carryover amplicons. In a preferred embodiment, the 3' exonuclease is selected from the group consisting of exonuclease I, exonuclease III (Exo III), exonuclease VIII, and ribonuclease II. In a most preferred embodiment the exonuclease is Exo III. Exonucleases are well known to the ordinarily skilled artisan and are commercially available.

It has been surprisingly discovered in accordance with the present invention that exonucleases that have been reported to be specific for double-stranded DNA nonetheless are capable of degrading single-stranded DNA primers. The degradation of single-stranded primers may be of such an extent as to reduce or eliminate amplification of the target sequence, thus leading to inefficient amplification or false negative results. The present method solves this problem by utilizing phosphorothioated primers in the PCR. Since phosphorothioated primers are protected from exonuclease digestion, the use of such primers in PCR allows the use of increased amounts of exonuclease per sample, and/or increased incubation times. In turn, the use of increased amounts of exonuclease and/or increased incubation times improves the effectiveness of nuclease pretreatment and thus provides further protection from the deleterious effects of primer-dimer molecules and PCR product carryover.

Phosphorothioated primers are oligonucleotide primers in which one or two of the nonbridging oxygens of the phosphate groups of a nucleotide is replaced by sulfur. The presence of phosphorothioate internucleotidic linkages in oligonucleotides result in inhibition of exonucleases, thus slowing or eliminating degradation of phosphorothioated primers. Phosphorothioated primers may be prepared by chemical or enzymatic methods well-known in the art, and disclosed for example by Eckstein (1985) Ann. Rev. Biochem. 54:367; Zon et al. (1991) Anti-Canc. Drug Des. 6:539; and Olsen et al. (1990) Proc. Natl. Acad. Sci. USA 87:1451, and in U.S. Patent No. 5,003,097. Phosphorothioated nucleotide derivatives are also available commercially.

One or more or all of the internucleotide linkages of the primers may be a phosphorothioate moiety. In a preferred embodiment, the oligonucleotide contains from one to four phosphorothioate linkages. Preferably at least one phosphorothioate linkage is at the ultimate, penultimate, third, fourth, of fifth position relative to the 3' terminus of the primer. The phosphorothioate linkages may be interspersed or adjacent. In a preferred embodiment, the linkages are adjacent and located at the 3' terminus of the primer. In another preferred embodiment, the primer contains a phosphorothioate linkage in at least the ultimate and penultimate positions relative to the 3' terminus of the primer. In another preferred embodiment, the primer contains a phosphorothioated linkage in at least the ultimate position relative to the 3' terminus of the primer. The ordinarily skilled artisan can determine the appropriate number and positions of phosphorothioate residues by assessing amplification in the presence of phosphorylated primers and a 3' exonuclease in a model system such as the system described in Examples 1 and 3.

In the method of the present invention, PCR amplification is accomplished by preincubating all PCR reagents and a sample containing a target nucleic acid in the presence of an exonuclease and phosphorothioated primers. The resulting reaction admixture is heated under conditions sufficient to inactivate the exonuclease, followed by formation and amplification of primer extension products.

The method of the present invention is particularly useful in PCR amplification and detection methods known in the art as homogeneous assays or homogeneous detection systems. Such systems are well-known in the art and are described, for example, in published European Patent Applications 91310062.4 (487218) and 92106989.4 (512334). In the homogeneous systems, detection of amplified DNA is based upon changes in fluorescence induced by binding of a fluorescent compound to double-stranded DNA. Because detection is based upon formation of double-stranded DNA generally, and fails to distinguish between target DNA and non-specific products, the formation of double-stranded artifacts such as primer-dimers is detrimental to the specificity of the homogeneous assay. In accordance with the present invention, primer-dimers are reduced and thus the specificity of the homogeneous detection method is increased.

The reagents required for PCR are known to the ordinarily skilled artisan, and generally include at least two oligonucleotide primers that are sufficiently complementary to conserved regions of the target nucleic acid to hybridize thereto, four different nucleoside triphosphates, a thermostable polymerization agent and any requisite cofactors for the polymerization agent. Preferred nucleoside triphosphates are the deoxyribonucleoside triphosphates dATP, dCTP, dGTP and dTTP or dUTP, collectively termed dNTPs. Nucleoside triphosphates are commercially available.

Primers include naturally occurring or synthetically produced oligonucleotides capable of annealing to the target nucleic acid and acting as the point of initiation of nucleic acid synthesis under appropriate conditions, i.e., in the presence of nucleoside triphosphates, a polymerization agent, suitable temperature, pH and buffer. The primers have sequences sufficiently complementary to the target nucleic acid to hybridize thereto, and are of sufficient length, typically from 10-60 nucleotides, to prime the synthesis of extension products in the presence of a polymerization agent. Primers may be produced synthetically by automated synthesis by methods well known to one of ordinary skill in the art. Synthesis of phosphorothioated primers is described hereinabove.

Design considerations for primers are well known in the art. Primers are selected to be substantially complementary to the sequences of the strands of the specific nucleic acid to be amplified, such that the extension product synthesized from one primer, when separated from its complement, can serve as a template for the extension product of the other primer. Preferably, the primers have exact complementarity with the target region.

Polymerization agents are compounds that function to accomplish the synthesis of the primer extension products. The polymerization agents are thermostable, i.e., not permanently inactivated when heated for brief periods to temperatures typically used in PCR for denaturation of DNA strands, e.g., 93-95°C, and are preferentially active at high temperatures. In a preferred embodiment the polymerization agent is a thermostable DNA polymerase, including, for example, DNA polymerase obtained from thermophilic bacteria such as, Thermococcus litoralis, Bacillus stearothermophilus, Methanothermus fervidus, Thermus aquaticus, T. filiformis, T. flavus, T. lacteus, T. rubens, T.ruber and T. thermophilus; or from thermophilic archaebacteria such as Desulfurococcus mobilis, Methanobacterium thermoautotrophilcum, Sulfolobus solfataricus*,* S. acidocaldarius and Thermoplasma acidophilum. In a most preferred embodiment, the polymerization agent is Thermus aquaticus (Taq) polymerase, T. thermophilus (Tth) polymerase or Thermococcus litoralis polymerase. Thermostable reverse transcriptase and DNA polymerases having reverse transcriptase activity are also contemplated as polymerization agents.

The thermostable polymerases may be obtained commercially or by methods known in the art. In particular, Taq polymerase is available commercially in recombinant and native form (Perkin Elmer-Cetus) or can be produced by the method described by Lawyer et al. (1989) J. Biol. Chem. 264:6427 or in U.S. Patent No. 4,889,818. Tth polymerase is commercially available from Finnzyme Co., Finland and from Toyobo Co., Japan. Thermococcus litoralis polymerase is commercially available from New England Biolabs and can be produced by the method described in U.S. Patent No. 5,322,785.

Antibodies specific for the thermostable polymerization agents may be included in the preamplification step to inhibit the polymerization agent prior to amplification. Antibodies can be produced by methods known to one of ordinary skill in the art and found, for example, in Harlowe et al. (1988) Antibodies: A Laboratory Manual, Cold Spring Harbor, NY. In accordance with the present invention, the term antibodies includes monoclonal and polyclonal antibodies produced by conventional methodologies, recombinantly produced antibodies, and chemically or recombinantly produced fragments of antibodies, such as Fab fragments. In a preferred embodiment, the antibodies are monoclonal.

In a preferred embodiment of the present invention, the antibody is a monoclonal antibody against Taq polymerase, Tth polymerase, or Thermococcus litoralis polymerase. In a more preferred embodiment, the antibody is a monoclonal antibody against Taq polymerase. Monoclonal antibodies against Taq polymerase are known in the art and described, for example, in U.S. Patent No. 5,338,671. In accordance with the present invention, antibodies defined as specific for polymerization agent are those antibodies that are capable of inhibiting the enzymatic activity of the polymerization agent at temperatures from about 20-40°C. The antibodies of the invention are inactivated by elevated temperatures used during PCR thermal cycling. The ability of the antibodies to inhibit enzymatic activity of the polymerase can be determined by assays known to one of ordinary skill in the art, as described, for example, by Sharkey et al. (1994) BioTechnology 12:506.

The exonucleases used in accordance with the present invention are commercially available or can be obtained by methods known in the art and include, for example, exonuclease III (Exo III), exonuclease I exonuclease VIII, and ribonuclease II. Exonucleases are known to the ordinarily skilled artisan, and are described, for example, by Fasman, ed. (1989) Practical Handbook of Biochemistry and Molecular Biology, CRC Press, BocaRaton, FL. 3' exonucleases are particularly contemplated in accordance with the present invention. Exonucleases that preferentially attack double-stranded DNA or single stranded DNA may be used. Exonucleases that preferentially attack double-stranded DNA are preferred. In a preferred embodiment, the exonuclease is Exo III. In another preferred embodiment, more than one exonuclease is utilized. For example, a mixture of an exonuclease that preferentially degrades double-stranded DNA, such as Exo III, can be used in combination with an exonuclease that preferentially degrades single-stranded DNA, such Exo I. Inactivation of the exonuclease at 95°C prevents further exonuclease activity during thermal cycling. Accordingly, the exonuclease must be inactive at 95°C.

The present invention provides a method for the amplification of a target nucleic acid, and optionally, the subsequent detection of the nucleic acid, in a sample suspected of containing the target nucleic acid. The sample may be any sample suspected of containing a target nucleic acid, including, for example, a tissue sample, blood, hair, body fluid, bacteria, virus, fungus, bacterial infected cell, virally infected cell, and so on. The target nucleic acid may be DNA or RNA. A sufficient number of bases at both ends of the sequence to be amplified must be known in order to design primers capable of hybridizing to the different strands of the target nucleic acid at suitable positions for PCR amplification. The target nucleic acid may be extracted or partially extracted from the tissue sample prior to PCR, for example, by removing proteins or cellular material from the sample. Methods for extracting nucleic acids from samples are known to one of ordinary skill in the art and may be found, for example, in Sambrook et al. (1989) Molecular Cloning: A Laboratory Manual, Cold Spring Harbor Laboratory Press, Cold Spring Harbor, NY and Saiki et al. (1985) BioTechnology 3:1008.

In the method of amplification of the present invention, the sample or a preparation of nucleic acids extracted from the sample is contacted with the reagents typically used for PCR, including at least two oligonucleotide primers modified to contain at least one phosphorothioate linkage, four different nucleoside triphosphates, a thermostable polymerization agent, and an appropriate buffer, and further with an exonuclease to form a reaction admixture. In another embodiment, an antibody specific for the polymerization agent is included in the admixture.

The conventional PCR reagents, including primers, nucleoside triphosphates, polymerization agent, and appropriate buffer are utilized at concentrations generally appropriate for PCR and known to one of ordinary- skill in the art. In a preferred embodiment, the nucleoside triphosphates are dATP, dCTP, dGTP and dTTP. In a preferred embodiment the polymerization agent is a thermostable DNA polymerase. Preferred DNA polymerases are Taq polymerase, Tth polymerase and Thermococcus litoralis polymerase. Taq polymerase is particularly preferred.

The antibody specific for the polymerization agent is used at a concentration effective to inhibit the polymerization agent at room temperature. The antibody may be monoclonal or polyclonal, or an antibody fragment. In a preferred embodiment, the antibody is monoclonal and is used at a molar ratio of from about 5 to about 500 over the polymerization agent. In a most preferred embodiment, the polymerization agent is Taq polymerase, the antibody is a monoclonal antibody specific for Taq polymerase.

Exonuclease may be used at a concentration of from about .001 Units/µL to about 10 Units/µL. In a preferred embodiment, the exonuclease is Exo III and is used at a concentration of from about .01 Units/µL to about .5 Units/µL. In a most preferred embodiment, Exo III is used at a concentration of 0.2 Units µL. One unit of Exo III is defined as that amount of Exo III required to produce 1 nanomole of said soluble nucleotides from tritiated DNA duplex in 30 minutes at 37°C. The skilled artisan can determine appropriate concentrations of exonuclease which may vary depending upon concentration of target and other experimental conditions.

Following contacting the sample with the reagents for PCR, phosphorothioated primers and exonuclease, and prior to thermal cycling, the reaction mixture is heated to inactivate exonuclease. In a preferred embodiment, the mixture is heated to about 85°C - 95°C for about three to about ten minutes. In a most preferred embodiment, the mixture is allowed to stand at room temperature for about zero to about eight hours, then incubated at about 40°C for up to about five minutes, then heated to about 85°C to about 95°C for at-least about three minutes.

Following heat denaturation, standard PCR cycling of annealing, extending and denaturing is performed. Cycling parameters are known to the ordinarily skilled artisan, and can be easily adapted for particular conditions. The amplification method is preferably conducted in a continuous, automated manner. Appropriate instrumentation for automated PCR is well-known to the ordinarily skilled artisan and described, for example, in U.S. Patent Nos. 4,965,188, 5,089,233 and 5,229,297. The skilled artisan can also easily detect amplified product, for example, by separating PCR products by agarose gel electrophoresis and visualizing by ethidium bromide staining, or detecting by hybridization with a labeled probe capable of hybridizing with the amplified nucleic acid or a variety of other detection methods well-known to one of ordinary skill in the art.

The present invention further provides a kit for PCR comprising, in the same or separate containers, a thermostable polymerization agent, an exonuclease, and at least two primers, each of which contains at least one phosphorothioate linkage. In another embodiment, the kit further comprises an antibody specific for the polymerization agent. Additional containers can also be provided for the inclusion of, for example, additional antibodies specific to the PCR polymerization agent and reagents for PCR, including, for example, nucleoside triphosphates, primers and buffers.

In a preferred embodiment the polymerization agent is a DNA polymerase. In a more preferred embodiment the polymerase is Taq polymerase, Tth polymerase, or Thermococcus litoralis polymerase. Taq polymerase is particularly preferred. The preferred antibody is a monoclonal antibody specific for Taq polymerase.

In another preferred embodiment, the exonuclease is exonuclease I, Exo III, exonuclease VIII or ribonuclease II. Exo III is particularly preferred. The oligonucleotide primers preferably contain a phosphorothioate linkage in at least one of the first five positions relative to the 3' terminus, and preferably in at least the ultimate position relative to the 3' terminus. The kits of the present invention are useful in increasing efficiency of amplification of target nucleic acids in PCR assays.

The present invention further provides an admixture useful for PCR amplification and particularly for reducing formation of non-specific nucleic acids and amplification of carryover contaminants during PCR. The admixture contains an exonuclease and at least two primers for PCR wherein each primer contains at least one phosphorothioate linkage. The admixture may also contain other PCR reagents such as a thermostable polymerization agent, nucleoside triphosphates, antibodies against the polymerization agent, buffers, and the like. In a preferred embodiment the reaction admixture comprises Exo III and at least two oligonucleotide primers wherein each of said oligonucleotide primers contains a phosphorothioate linkage in at least the ultimate position relative to the 3' terminus of the primer.

The following examples further illustrate the present invention.

### EXAMPLE 1

### Exonuclease III Effects on PCR

The effect of a relatively low concentration of Exo III on PCR amplification of a genomic target was examined in a model system for the detection of HIV. The model system utilizes primers SK38 and BW17 having the following sequences: 
Primer SK38: 5'-ATAATCCACC TATCCCAGTA GGAGAAAT-3' (SEQ ID NO:1)
Primer BW17: 5'-TTTGGTCCTT GTCTTATGTC CAGAATGC-3' (SEQ ID NO:2)
 for the detection of HIV target obtained from the 8E5 HUT/HIV cell line containing a single integrated copy of the HIV-1 genome. Oligonucleotide primers were prepared by solid phase phosporamidite chemistry utilizing a Perkin-Elmer/Applied Biosystems Division Model 380B three column DNA synthesizer according to U.S. Patent No. 5,003,097. Monoclonal antibodies specific for Taq polymerase were prepared as described in U.S. Patent No. 5,338,671. Taq polymerase was prepared recombinantly as described in EP-A 0 482 714.

The PCR mix contained the following reagents:
(1) 1.0 X PCR buffer (10 mM Tris Hcl, pH 8.0 with 50 mM KCl and 5 mM Mgcl₂);
(2) SK38 PCR primer;
(3) BW17 PCR primer;
(4) dATP, dCTP and dGTP at .2 mM each;
(5) dUTP at .4 mM;
(6) Taq polymerase at 8 units per 100 µL PCR mix (one unit is defined as the amount of enzyme activity required to incorporate 10 nmoles of total nucleotides into an extending nucleic acid chain in 30 minutes at 74°C);
(7) Taq antibodies at 5:1 (TP4-9.2 ATCC HB11807) and 50:1 (TP1-12.2 ATCC HB11127) Molar ratio over Taq polymerase;
(8) Herring Sperm DNA at 1 µG per 100 µL PCR mix;
(9) 100 copies of HIV (8E5 HUT/HIV cell line);
(10)TE buffer (10 mM Tris • HCl, 1 mM EDTA)

Individual PCR primers were each used at the following levels:
(1) 400 nM;
(2) 100 nM;
(3) 40 nM;
(4) 20 nM;
(5) 10 nM;
(6) 4 nM;
(7) 1 nM;
(8) .4 nM.

PCR was performed in the presence or absence of exonuclease III (Promega, Lot #32000) at .02 units per µL of PCR mix [2 units per 100 µL PCR mix) at the primer levels described above.

PCR amplification was performed in the Clinical Diagnostics PCR pouch described in U.S. Patent No. 5,229,297 utilizing the Prototype Analyzer described in U.S. Patent No. 5,089,233. All samples were run in duplicate and left out at room temperature for 2 hours prior to PCR to allow exonuclease III to be active in the PCR mix. Following the 2 hour incubation samples underwent standard PCR cycling conditions consisting of a 3 minute 95°C preheat for exonuclease III and Taq antibody thermal denaturation before 40 cycles of PCR (95°C 15 second melting step and 64°C 35 second annealing/extending step per cycle). The PCR product was detected using the Clinical Diagnostic's Pouch detection system. The detection spots were visually read using a color standard that increases in blue color from 0 (white minimal or no amplification) to 10 (dark blue maximum amplification). The visual results are in tabular form below comparing color scores of samples +/- exonuclease III (at 2 units for 100 µL of PCR mix):

| **Primer Level** | **+ Exo III** | **- Exo III** |
|---|---|---|
| 400 nM | 7, 6.5 | 7, 6.5 |
| 100 nM | 0, 0 | 7, 7.5 |
| 40 nM | 0, 0 | 7, 6.5 |
| 20 nM | 0, 0 | 2, 4 |
| 10 nM | 0, 0 | 0, 0 |
| 4 nM | 0, 0 | 0, 0 |
| 1 nM | 0, 0 | 0, 0 |
| .4 nM | 0, 0 | 0, 0 |

A 4-fold decrease in primer concentration causes negative results in samples with exonuclease III while a 10-fold decrease in primer concentration has no effect on samples without exonuclease III. A 20-fold decrease in primer concentration still gives PCR amplification in samples without exonuclease III. It is clear from these results that exonuclease III has a negative effect on PCR amplification as the primer concentration in the PCR mix is decreased. It can be assumed from this result that the amplification efficiency of PCR is therefore decreased in samples with exonuclease III, perhaps even at 400 nM primer concentrations.

### EXAMPLE 2

### Exo III Degrades Double-Stranded and Single-Stranded DNA

The possibility that exonuclease III can degrade single-stranded DNA, contrary to the manufacturer's claims and the relevant scientific literature (Zhu et al., (1991) Nucleic Acids Res. 19:251), was investigated.

Single-stranded DNA targets, double-stranded DNA targets, and single-stranded oligonucleotide primers, alone or in various combinations, were subjected to a pre-amplification sterilization procedure in the presence or absence of exonuclease III.

The single-stranded 100mer DNA target, a synthetic model system designated Syn-1, was used at a concentration of 1 x 10⁻¹⁰ M. The Syn-1 target has the sequence:

5′-AAT-CGA-GTA-AGA-CTT-CAC-TGC-TGA-GAA-
TCT-CAG-AGA-ATC-TAG-ATA-TCC-TGC-ATG-TCT-
AAA-TAT-TGA-ATA-CGA-CAT-TAC-ACG-AGT-CAA-
GAC-TCA-CTA-GAC-A-3′ (SEQ ID NO:3)

The double-stranded target, the HIV model system described in Example 1, was used at a concentration of 1 x 10⁻⁸ M. PCR primers Syn-1 forward,

5′-GTA-AGA-CTT-CAC-TGC-TGA-GAA-TCT-CAG-3′  (SEQ ID NO:4)

Syn-1 reverse, 5′-TGT-CTA-GTG-AGT-CTT-GAC-TCG-TGT-AAT-3′ (SEQ ID NO:5) SK38 and BW17 were used at a total concentration of 2 µM (i.e., 2 µM primer for samples containing one primer, 1 µM each for samples containing two primers). Exonuclease III (Promega, Lot #32000) was used at 0, 10 or 20 units per 50 µL sample.

Samples were incubated under typical preamplification sterilization conditions (30°C for 30 minutes). Enzyme-mediated hydrolysis was quenched by a five minute incubation at 95°C to thermally denature exonuclease III. Small aliquots of the reaction mixtures (16 µL) were electrophoresed on a 4% agarose gel under standard conditions and visualized by ethidium bromide staining. The results observed on the gel are presented in Table I.

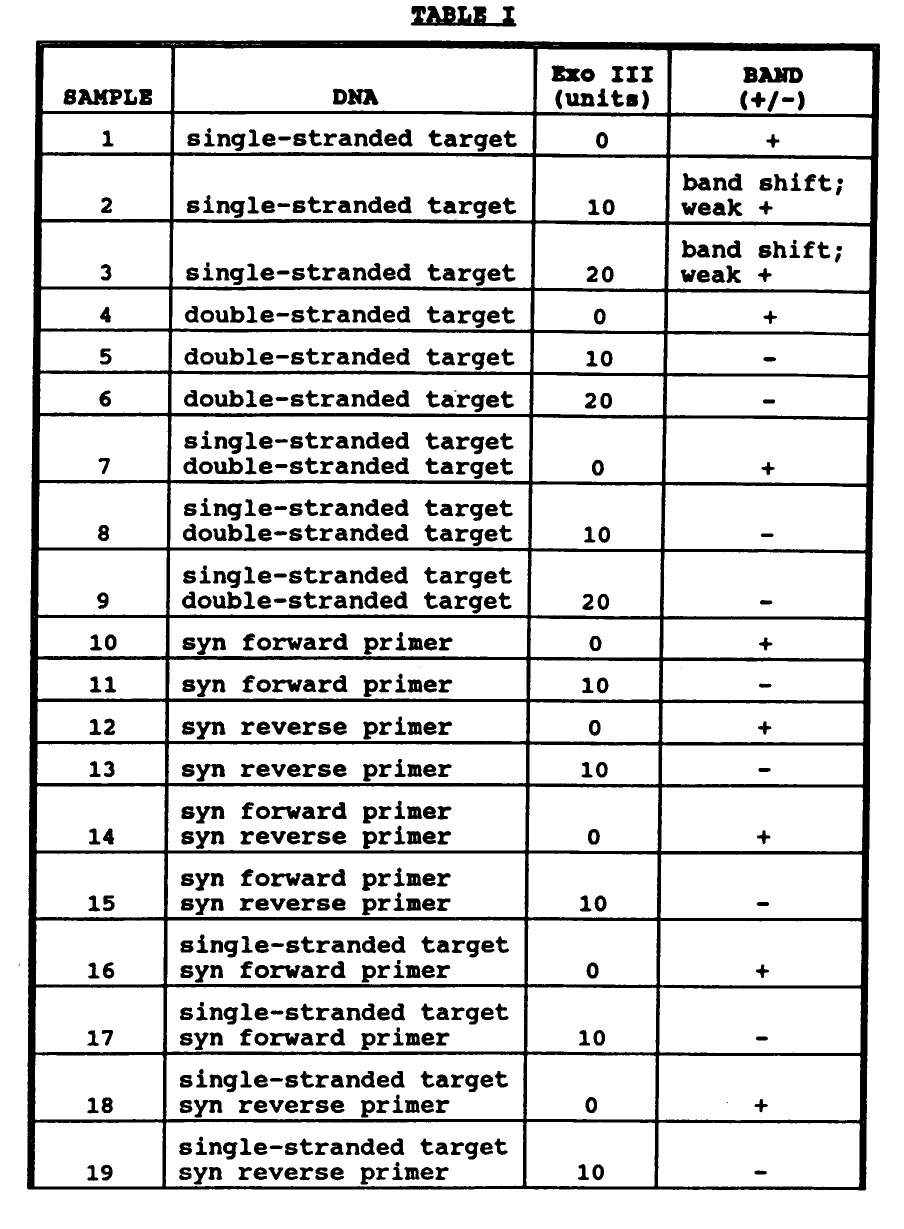

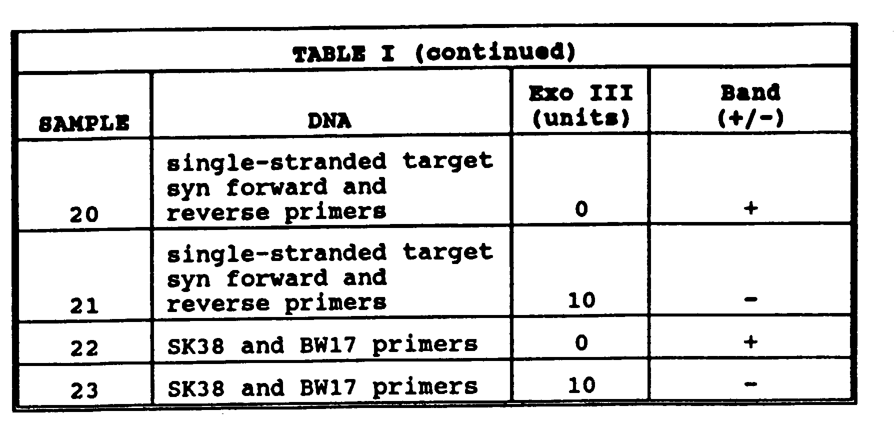

In Samples 2 and 3, the target bands were shifted down on the gel relative to Sample 1, indicating that the molecular weight of the single-stranded target was reduced by Exo III degradation. The signal of the target band of Samples 2 and 3 was also reduced relative to Sample 1, further indicating that the single-stranded target was degraded by Exo III.

This experiment demonstrates that Exo III degrades both single-stranded and double-stranded DNA.

The positive controls (unreacted with exonuclease III) provided strong signal bands in all sample types. Except for Samples 2 and 3, all DNA species treated with exonuclease III at 10 or 20 units per 50 µL sample (single-stranded DNA, duplex DNA, short PCR primers and 100-mer target sequences; duplex DNA was a PCR product of the SK18/BW17 system) were degraded and rendered undetectable on the electrophoresis gel. This single-stranded DNA degradation by exonuclease III provides an explanation for the negative effect of exonuclease III on PCR as seen in Example 1. The destruction of PCR primers, a critical reagent in the PCR reaction, reduces primer concentration and thus the amplification efficiency of PCR.

### EXAMPLE 3

### Phosphorothioated Primers Provide Protection Against Exonuclease III Degradation

The SK38/BW17 HIV PCR model system described in Example 1 was used in this experiment.
Phosphorothioated primers were substituted for unmodified primers in PCR to determine whether the modified primers provide protection against exonuclease III degradation. Parallel samples were run in the absence of target DNA to rule out target DNA degradation by exonuclease III.

PCR primers SK38 and BW17 having phosphorothioate linkages in the ultimate and penultimate positions relative to the 3'-hydroxyl group were prepared by H-phosphonate chemistry according to the method of U.S. Patent No. 5,003,097, also described in the technical bulletin accompanying Cat. No. 40-4036-xx, Glen Research, Sterling, Va.
All samples contained the following reagents: 1.0X PCR buffer with 5 mM magnesium;
SK38 PCR primer, unmodified (SOA) or phosphorothioated (thio), at 400 nM;
BW17 PCR primer, unmodified (SOA) or phosphorothioated (thio), at 400 nM;
dATP, dCTP and dGTP at .2 mM each;
dUTP at .4 mM;
Taq polymerase at 8 units per 100 µL PCR mix;
Taq antibodies at 5:1 (TP4-9.2) and 50:1 (TP1-12.2) molar ratio over Taq polymerase;
herring sperm DNA at 1 µg per 100 µL PCR mix;
TE buffer.

Samples 1-10 contained 10 copies of HIV genomic target obtained from the 8E5 HUT/HIV cell line.

The samples contained various concentrations of exonuclease III (0, 5, 10, 15, 20 units per 100 µL PCR mix) as indicated in Table II.

The Clinical Diagnostic's PCR Pouch and Prototype Analyzer were used for PCR amplification. All samples were run in duplicate and left out at room temperature for 1 hour prior to PCR to allow exonuclease III to be active in the PCR mix. In samples 11-18, genomic target DNA was added to the mix following the 1 hour incubation with exonuclease III. Following the 1 hour incubation, samples underwent standard PCR cycling conditions consisting of a 3 minute 95°C preheat for exonuclease III and Taq antibody thermal denaturation before 40 cycles of PCR (95°C 15 second melting step and 64°C 35 second annealing/ extending step per cycle). Following PCR amplification, one replicate per sample underwent detection using the Clinical Diagnostic's Pouch detection system. The detection spots were visually read using a color standard that increases in blue color from 0 (white) to 10 (dark blue). The PCR product from the other replicate was collected from the PCR pouch and detected by electrophoresis on a 2.5% agarose gel stained with ethidium bromide. The gel was illuminated with UV light and a Polaroid picture was taken.

**TABLE II**

| **SAMPLE** | **PRIMER** | **TARGET*** | **EXO III (units/100 µL)** | **POUCH** | **GEL** |
|---|---|---|---|---|---|
| 1 | SOA | b | 0 | 7 | weak positive |
| 2 | SOA | b | 5 | 0 | negative |
| 3 | SOA | b | 10 | 0 | negative |
| 4 | SOA | b | 15 | 0 | negative |
| 5 | SOA | b | 20 | 0 | negative |
| 6 | Thio | b | 0 | 6.5 | very weak positive |
| 7 | Thio | b | 5 | 6 | strong positive |
| 8 | Thio | b | 10 | 6.5 | strong positive |
| 9 | Thio | b | 15 | 6.5 | strong positive |
| 10 | Thio | b | 20 | 6.5 | strong positive |
| 11 | SOA | a | 5 | 0 | negative |
| 12 | SOA | a | 10 | 0 | negative |
| 13 | SOA | a | 15 | 0 | negative |
| 14 | SOA | a | 20 | 0 | negative |
| 15 | Thio | a | 5 | 7 | strong positive |
| 16 | Thio | a | 10 | 7 | strong positive |
| 17 | Thio | a | 15 | 7 | strong positive |
| 18 | Thio | a | 20 | 6.5 | strong positive |

| | | | | | |
|---|---|---|---|---|---|
| * a, target added after Exo III incubation; b, target added before Exo III incubation. | | | | | |

The results for pouch detection and gel visualization are presented in Table If. The results from Samples 2-5 indicate that exonuclease III renders PCR amplification undetectable when standard primers are used. The negative effect of exonuclease III on PCR can be attributed to degradation of unmodified PCR primers, as opposed to degradation of genomic target DNA, as evidenced by the negative results in Samples 11-14, in which target DNA was added after exonuclease III incubation.

As evidenced by the results of Samples 6-10 and 15- 18, phosphorothioated PCR primers do not inhibit PCR, but rather are readily amplified and thus can be used routinely. Further, phosphorothioated PCR primers are protected against exonuclease III degradation, whereas unmodified primers are not.

The use of exonuclease III in combination with phosphorothioated PCR primers results in an enhancement in product yield as evidenced by gel band intensity in Samples 7-10 in comparison to Samples 1 and 6. These results can be interpreted that PCR amplification efficiency is increased by the ability of exonuclease III to degrade PCR primer-dimers prior to amplification, and also due to the protection of phosphorothioated PCR primers against exonuclease III.

### EXAMPLE 4

### Low Levels of Genomic Target DNA are not Degraded by Exonuclease III

The SK38/BWl17 HIV model system described in Example 1 was used in this experiment. Two levels of genomic target (10 copies and 100 copies of HIV genomic target from the 8E5 HUT/HIV cell line) were amplified in the presence of various concentrations of exonuclease III and either unmodified (SOA) or phosphorothioated (thio) primers as described in Example 3.

All samples contained the following reagents:
1.0X PCR buffer with 5 mM magnesium;
SK38 PCR primer, unmodified (SOA) or phosphorothioated as described in Example 3 (thio), at 400 nM;
BW17 PCR primer, unmodified (SOA) or phosphorothioated as described in Example 3 (thio), at 400 nM;
dATP, dCTP and dGTP at .2 mM each;
dUTP at .4 mM;
Taq polymerase at 8 units per 100 µL PCR mix;
Taq antibodies at 5:1 (TP4-9.2) and 50:1 (TP1-12.2) molar ratio over Taq polymerase;
herring sperm DNA at 1 µg per 100 µL PCR mix;
TE buffer.
Samples 1-16 contained 10 copies of HIV target.
Samples 17-32 contained 100 copies of HIV target. Samples 1-8 and 17-24 contained unmodified (SOA) primers and Samples 9-16 and 25-32 contained phosphorothioated (thio) primers. The samples contained various concentrations of exonuclease III as indicated in Table III.

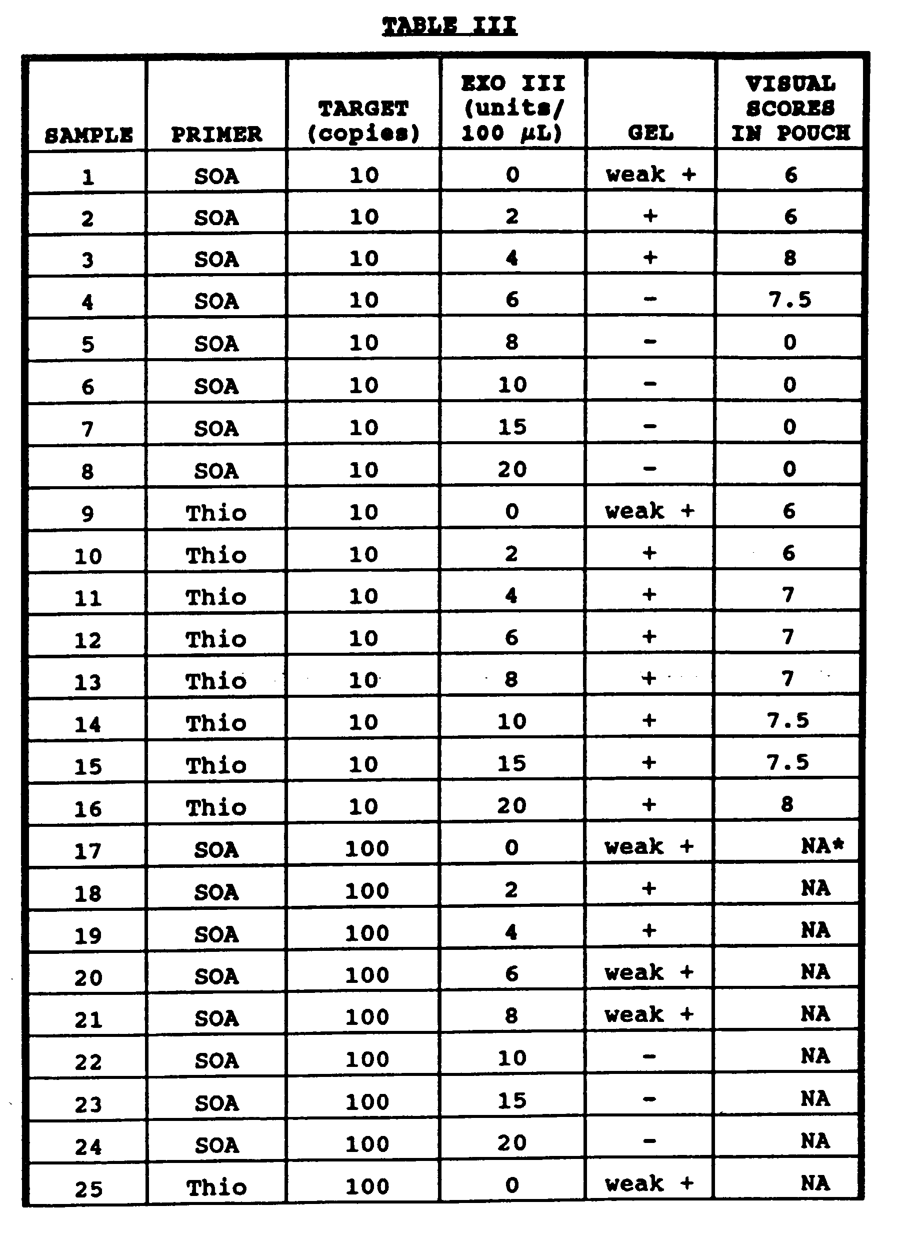

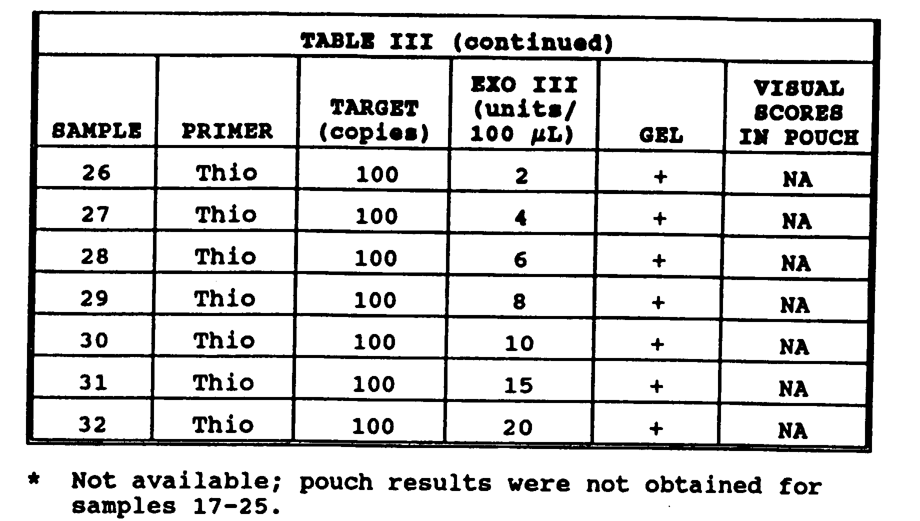

The Clinical Diagnostic's PCR Pouch and Prototype Analyzer were used for PCR amplification. All samples were run in duplicate and left out at room temperature for 2 hours prior to PCR to allow exonuclease III to be active in the PCR mix. Following the 2 hour incubation, samples underwent standard PCR cycling conditions consisting of a 3 minute 95°C preheat for exonuclease III and Taq antibody thermal denaturation before 40 cycles of PCR (95°C 15 second melting step and 64°C 35 second annealing/extending step per cycle). Following PCR amplification, one replicate per each sample underwent detection using the Clinical Diagnostic's Pouch detection system. The detection spots were visually read using a color standard that increases in blue color form 0 (white) to 10 (dark blue). The PCR product from the other replicate was collected from the PCR pouch and detected by electrophoresis on a 2.5% agarose gel stained with ethidium bromide. The gel was illuminated with UV light and a Polaroid picture was taken.

Results are presented in Table III. These results confirm the results of Example 2, i.e., that exonuclease III degrades unmodified single-stranded PCR primers such that products are not detectable, and that phosphorothioated PCR primers are protected against exonuclease III digestion. Accordingly, the use of phosphorothioated primers allows the use of increased amounts of exonuclease III per sample, and increased incubation times. In turn, increased amounts of exonuclease III offer more protection against the inhibitory effects of primer-dimer molecules and against PCR carryover in the form of PCR product and primer-dimer.

An enhancement in product yield, as evidenced by gel band intensity, is seen in samples that contain phosphorothioated PCR primers and exonuclease III at all concentrations of exonuclease III tested, and in samples that contain SOA unmodified primers and exonuclease III at 2 or 4 units per 100 µL PCR mix in comparison to samples without exonuclease III for both primer types. This is due to exonuclease III's ability to degrade PCR primer-dimer molecules before PCR takes place, enhancing the PCR amplification efficiency of the PCR reaction. However, in samples with SOA unmodified PCR primers, exonuclease III becomes an inhibitor of PCR at slightly higher levels due to its single-strand DNA degradation activity.

Genomic target DNA, even low copy numbers per sample, is not degraded by exonuclease III at elevated levels as evidenced by the consistent PCR pouch color scores and gel product bands for the 10 copy HIV samples in which phosphorothioated primers are used.

### EXAMPLE 5

### PCR Carryover Sterifisation with a Combination of Exonuclease III and Phosphorothioated Primers

This experiment determined the sterilization efficiency of exonuclease III at 20 units per 100 µL of PCR mix with phosphorothioated PCR primers. Further, the ability of exonuclease III and phosphorothioated primers to sterilize PCR amplicons was compared with uracil-N-glycosylase (UNG), which has been reported to have a sterilization capacity of about 10⁶ amplicons (Longo et al.).

The SK38/BW17 HIV PCR model system was used with PCR carryover collected from an earlier amplification. The PCR carryover was a preamplified PCR product at full strength estimated to be at a concentration of 10¹¹ amplicons per µL and in which dUTP was substituted for TTP during amplification so that the product contains uracil in place of thymidine. This preamplified product was run on a 4% agarose electrophoresis gel to isolate the product from nonspecific products. The product band was then cut out of the agarose gel and purified using Qiagen's Gel Purification Kit. Dilutions of this purified PCR product were than made with the following estimates of PCR carryover amplicons per µL per dilution:
(1) 10x dilution - 10¹⁰ amplicons per µL;
(2) 10³x dilution = 10⁸ amplicons per µL;
(3) 10⁵x dilution = 10⁶ amplicons per µL;
(4) 10⁷x dilution = l0⁴ amplicons per µL;
(5) 10⁹x dilution = 10² amplicons per µL;

One µL of each carryover dilution was added to 100 µL of PCR mix that contained no genomic target (except for the positive control samples which contained 10 copies of Genomic HIV target per 100 µL of PCR mix) and the following standard PCR reagents:
1.0X PCR buffer with 5 mM magnesium;
SK38 PCR primer (phosphorothioated as described in Example 3);
BW17 PCR primer (phosphorothioated as described in Example 3);
dATP, dCTP and dGTP at .2 mM each;
dUTP at .4 mM;
Taq polymerase at 8 units per 100 µL PCR mix;
Taq antibodies at 5:1 (TP4-9.2) and 50:1 (TP1-12.2) molar ratio over Taq polymerase;
Herring sperm DNA at 1 µG per 100 µL PCR mix;
TE buffer.

Samples were tested with no sterilization reagents, or with Exo III (20 units per 100 µL PCR mix, obtained from Promega, Lot #32000) or UNG (1 unit per 100 µL PCR mix, obtained from Perkin Elmer Applied Biosystems, Lot #0642), alone or in combination, as indicated in Table IV.

**TABLE IV**

| **SAMPLE** | **TARGET (copies 100µL)** | **AMPLICONS** | **EXO III** | **UNG** | **POUCH COLOR SCORE** |
|---|---|---|---|---|---|
| 1 | 10 | 0 | - | - | 6.5 |
| 2 | 10 | 0 | + | + | 6 |
| 3 | 0 | 10² | - | - | 5 |
| 4 | 0 | 10² | + | - | 0 |
| 5 | 0 | 10² | - | + | 0 |
| 6 | 0 | 10² | + | + | 0 |
| 7 | 0 | 10⁴ | - | - | 5.5 |
| 8 | 0 | 10⁴ | + | - | 0 |
| 9 | 0 | 10⁴ | - | + | 0 |
| 10 | 0 | 10⁴ | + | + | 0 |
| 11 | 0 | 10⁶ | - | - | 6 |
| 12 | 0 | 10⁶ | + | - | 5 |
| 13 | 0 | 10⁶ | - | + | 6 |
| 14 | 0 | 10⁶ | + | + | 6 |
| 15 | 0 | 10⁸ | - | - | 6 |
| 16 | 0 | 10⁸ | + | - | 6 |
| 17 | 0 | 10⁸ | - | + | 5 |
| 18 | 0 | 10⁸ | + | + | 6.5 |
| 19 | 0 | 10¹⁰ | - | - | 6.5 |
| 20 | 0 | 10¹⁰ | + | - | 6.5 |
| 21 | 0 | 10¹⁰ | - | + | 6.5 |
| 22 | 0 | 10¹⁰ | + | + | 6.5 |

The Clinical Diagnostic's PCR Pouch and Prototype Analyzer were used for PCR amplification. All samples were left out at room temperature for 2 hours prior to PCR to allow exonuclease III and UNG to be active in the PCR mix. Following the 2 hour incubation, samples underwent a preheat of 10 minutes at 95°C for the thermal denaturation of UNG, exonuclease III, and Taq antibody. Standard PCR cycling conditions were then used for 40 cycles of PCR (95°C 15 second melting step and 64°C 35 second annealing/extending step per cycle). Following PCR amplification, each sample underwent detection using the Clinical Diagnostic's Pouch's detection system. The detection spots were visually read using a color standard that increases in blue color from 0 (white) to 10 (dark blue). The results for each sample are presented in Table IV.

These results demonstrate that exonuclease III can sterilize at least 10,000 amplicons at 20 units per 100 µL of a PCR mix containing phosphorothioated primers. This method of sterilization was comparable to the level achieved with UNG, indicating that phosphothioated primers do not interfere with sterilization.

### EXAMPLE 6

### Comparison of Effectiveness of UNG and Exo III at Eliminating Primer - Dimer Carryover

Primer-dimer carryover was obtained from an amplified mix containing SK38/B%l17 primer but no target, i.e. conditions favoring primer-dimer formation, and added to PCR samples prior to the room temperature incubation at varying dilutions from 1 x 10 to 1 x 10⁸. The primer-dimer generated in this way was too weak to be visible as a band on an ethidium stained gel, but its presence was clearly mainfest, as the following makes clear. The PCR mix contained the reagents as described in Example 1 except that phosphorthioated primers were used. All samples except Sample 1 contained 100 copies of HIV genomic target obtained from the 8E 5 HUT/HIV cell line. Sample 2 contained no carryover, and Sample 3 was exposed to environmental carryover. Samples were tested in triplicate under the following conditions: A) No carryover prevention methods; B) UNG (Perkin-Elmer lot #0642), at 1 Unit/100µL PCR reaction; and C) Exo III at 20 units/100µL PCR reaction. Phosphorothioated primers were used in all case. Samples in Group A were exposed to a ten minute incubation at room temperature prior to standard PCR cycling. Samples in Group B were incubated for ten minutes at room temperature prior to a two minute incubation at 50°C, followed by standard PCR cycling followed by holding at 72°C. Samples in Group C were incubated for two hours at room temperature followed by standard PCR cycling. Aliquots of each sample were electrophoresed on a 4% agarose gel under standard conditions and visualized by ethidium bromide staining. The following results were observed.

| **Group A - No Treatment** | | | |
|---|---|---|---|
| **SAMPLE** | | **PRODUCT** | **PRIMER-DIMER** |
| 1 | no target | - | - |
| 2 | no carryover | strong + | very weak + |
| 3 | environmental carryover | weak + | + |
| 4a | 10X | - | strong + |
| 4b | 10X | - | strong + |
| 4c | 10X | - | strong + |
| 5a | 10³X | - | strong + |
| 5b | 10³X | - | strong + |
| 5c | 10³X | - | strong + |
| 6a | 10⁴X | - | strong + |
| 6b | 10⁴X | - | strong + |
| 6c | 10⁴X | - | strong + |
| 7a | 10⁵X | - | strong + |
| 7b | 10⁵X | - | strong + |
| 7c | 10⁵X | - | strong + |
| 8a | 10⁶X | - | strong + |
| 8b | 10⁶X | - | strong + |
| 8c | 10⁶X | - | strong + |
| 9a | 10⁷X | - | strong + |
| 9b | 10⁷X | - | strong + |
| 9c | 10⁷X | - | strong + |
| 10a | 10⁸X | - | strong + |
| 10b | 10⁸X | - | strong + |
| 10c | 10⁸X | - | strong + |
| * numerals indicate dilution of primer-dimer carryover | | | |

| **Group B - UNG Treatment** | | | |
|---|---|---|---|
| **SAMPLE** | | **PRODUCT** | **PRIMER-DIMER** |
| 1 | no target | - | - |
| 2 | no carryover | strong + | - |
| 3 | environmental carryover | + | + |
| 4a | 10X | - | strong + |
| 4b | 10X | - | strong + |
| 4c | 10X | - | strong + |
| 5a | 10³ | - | strong + |
| 5b | 10³ | - | strong + |
| 5c | 10³ | - | strong + |
| 6a | 10⁴ | - | strong + |
| 6b | 10⁴ | - | strong + |
| 6c | 10⁴ | - | strong + |
| 7a | 10⁵ | - | strong + |
| 7b | 10⁵ | - | strong + |
| 7c | 10⁵ | - | strong + |
| 8a | 10⁶ | weak + | weak + |
| 8b | 10⁶ | weak + | weak + |
| 8c | 10⁶ | weak + | weak + |
| 9a | 10⁷ | + | + |
| 9b | 10⁷ | weak + | + |
| 9c | 10⁷ | + | weak + |
| 10a | 10⁸ | weak + | + |
| 10b | 10⁸ | weak + | + |
| 10c | 10⁸ | weak + | + |

| **Group C - Exo III Treatment** | | | |
|---|---|---|---|
| **SAMPLE** | | **PRODUCT** | **PRIMER-DIMER** |
| 1 | no target | - | - |
| 2 | no carryover | + | - |
| 3 | environmental carryover | + | - |
| 4a | 10X | - | + |
| 4b | 10X | - | + |
| 4c | 10X | - | + |
| 5a | 10³X | weak + | weak + |
| 5b | 10³X | weak + | weak + |
| 5c | 10³X | weak + | weak + |
| 6a | 10⁴X | + | - |
| 6b | 10⁴X | + | - |
| 6c | 10⁴X | + | - |
| 7a | 10⁵X | + | - |
| 7b | 10⁵X | + | - |
| 7c | 10⁵X | + | - |
| 8a | 10⁶X | + | - |
| 8b | 10⁶X | + | - |
| 8c | 10⁶X | + | - |
| 9a | 10⁷X | + | - |
| 9b | 10⁷X | + | - |
| 9c | 10⁷X | + | - |
| 10a | 10⁸X | + | - |
| 10b | 10⁸X | + | - |
| 10c | 10⁸X | + | - |

With UNG treatment, product bands were detected at a 1 X 10⁶ dilution of the primer-dimer carryover, representing a 1000X improvement over no treatment. (Previous experiments have demonstrated that in the absence of treatment, product bands were detectable at a 1 X 10¹⁰ dilution of primer-dimer carryover.) With Exo III treatment in the presence of thioated primers, product bands were detected at a 1 X 10³ dilution of primer-dimer carryover, representing a 1000X improvement over UNG treatment and a 1 X 10⁷ improvement compared to the absence of treatment. Accordingly, Exo III is superior to UNG or absence of treatment at eliminating primer-dimer carryover.

## Claims

1. A method for the amplification of a target nucleic acid comprising:
(a) contacting a sample suspected of containing said target nucleic acid with (i) at least two oligonucleotide primers that are sufficiently complementary to regions of said target nucleic acid to hybridize thereto, wherein each of said oligonucleotide primers contain at least one phosphorothioate linkage, (ii) at least four different nucleoside triphosphates, (iii) a thermostable polymerization agent, and (iv) at least one 3' exonuclease to form a reaction mixture;
(b) heating said reaction admixture to inactivate said exonuclease; and
(c) forming primer extension products.

2. The method of claim 1 wherein said target nucleic acid is DNA or RNA.

3. The method of claim 1 or claim 2 wherein said nucleoside triphosphates are dATP, dGTP, dCTP and dTTP.

4. The method of any one of claims 1 to 3 wherein said polymerization agent is a DNA polymerase such as Thermus aquaticus (Taq) polymerase, Thermus thermophilus polymerase or Thermococcus litoralis polymerase.

5. The method of any one of claims 1 to 4 further comprising contacting said sample with an antibody specific to said polymerization agent to form said reaction admixture.

6. The method of claim 5 wherein said polymerization agent is Taq polymerase and said antibody is a monoclonal against Taq polymerase.

7. The method of any one of claims 1 to 6 wherein said 3' exonuclease is exonuclease I, exonuclease III, exonuclease VIII, or ribonuclease II.

8. The method of claim 7 wherein said 3' exonuclease is exonuclease III which preferably is present at a concentration of from about .001 units to about 10 units per microliter of reaction admixture, most preferably about 0.2 units per microliter of reaction admixture.

9. The method of any one of claims 1 to 8 wherein each of said primers contains a phosphorothioate linkage in at least the ultimate position and optionally also in the penultimate position relative to the 3' terminus of the primer.

10. The method of any one of claims 1 to 9 wherein said reaction mixture comprises two 3' exonucleases.

11. The method of claim 10 wherein one of said 3' exonucleases preferentially degrades doubled-stranded DNA and one of said 3' exonucleases preferentially degrades single-stranded DNA.

12. The method of any one of claims 1 to 11 wherein said heating is from about 85°C to about 95°C.

13. The method of any one of claims 1 to 12 which further comprises detecting primer extension products, preferably by measuring fluorescence changes induced by binding of a fluorescent compound to double-stranded DNA.

14. A kit for amplification of a target nucleic acid comprising, in the same or separate containers, a thermostable polymerization agent, at least one 3' exonuclease, and at least two oligonucleotide primers that are sufficiently complementary to regions of said nucleic acid to hybridize thereto, wherein each of said oligonucleotide primers contains at least one phosphorothioate linkage.

15. The kit of claim 14 further comprising in the same or a separate container, an antibody specific to said polymerization agent.

16. The kit of claim 14 or claim 15 wherein said polymerization agent is Taq polymerase, Thermus thermophilus polymerase, or Thermococcus litoralis polymerase.

17. The kit of any one of claims 14 to 16 wherein said exonuclease is exonuclease I, exonuclease III, exonuclease VIII or ribonuclease II and is preferably exonuclease III.

18. The kit of any one of claims 14 to 17 wherein each of said primers contains a phosphorothioate linkage in at least the ultimate position and optionally also in the penultimate position relative to the 3' terminus of said primer.

19. An admixture for use in PCR comprising at least one 3' exonuclease and at least two oligonucleotide primers wherein each of said oligonucleotide primers contains at least one phosphorothioate linkage.

20. The admixture of claim 19 further comprising a thermostable polymerization agent which is preferably a DNA polymerase such as Thermus aquaticus (Taq) polymerase, Thermus thermophilus polymerase or Thermococcus litoralis polymerase.

21. The admixture of claim 19 or claim 20 further comprising at least four different nucleoside triphosphates.

22. The admixture of any one of claims 19 to 21 wherein said exonuclease is exonuclease I, exonuclease III, exonuclease VIII, or ribonuclease II.

23. An admixture for use in PCR comprising exonuclease III and at least two oligonucleotide primers wherein each of said oligonucleotide primers contains a phosphorothioate linkage in at least the ultimate position relative to the 3' end of the primer.


SEQUENCE LISTING

(1) GENERAL INFORMATION:

(i) APPLICANT: Backus, John W.
Patterson, David R.
Sutherland, John W. H.

(ii) TITLE OF INVENTION: METHODS FOR POLYMERASE CHAIN
REACTION PREAMPLIFICATION STERILIZATION BY
EXONUCLEASES IN THE PRESENCE OF PHOSPHOROTHIOATED
PRIMERS

(iii) NUMBER OF SEQUENCES: 5

(iv) CORRESPONDENCE ADDRESS:
(A) ADDRESSEE: Johnson & Johnson
(B) STREET: One Johnson and Johnson Plaza
(C) CITY: New Brunswick
(D) STATE: New Jersey
(E) COUNTRY: U.S.A.
(F) ZIP: 08933

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Ciamporcero, Audley A.
(B) REGISTRATION NUMBER: 26,051
(C) REFERENCE/DOCKET NUMBER: CDS-19

(ix) TELECOMMUNICATION INFORMATION:
(A) TELEPHONE: (908) 524-2803
(B) TELEFAX: (908) 524-2808

(2) INFORMATION FOR SEQ ID NO:1:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

ATAATCCACC TATCCCAGTA GGAGAAAT 28

(2) INFORMATION FOR SEQ ID NO:2:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

TTTGGTCCTT GTCTTATGTC CAGAATGC 28

(2) INFORMATION FOR SEQ ID NO:3:

(i) SEQUENCE CHARACTERISTICS:
(A) LENGTH: 100 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

AATCGAGTAA GACTTCACTG CTGAGAATCT CAGAGAATCT AGATATCCTG CATGTCTAAA 60

TATTGAATAC GACATTACAC GAGTCAAGAC TCACTAGACA 100

(2) INFORMATION FOR SEQ ID NO:4:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

GTAAGACTTC ACTGCTGAGA ATCTCAG 27

(2) INFORMATION FOR SEQ ID NO:5:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

TGTCTAGTGA GTCTTGACTC GTGTAAT 27